Europäisches Patentamt

European Patent Office

Office européen des brevets

(11) Numéro de publication: **0 366 515 B1**

## (12) FASCICULE DE BREVET EUROPEEN

(45) Date de publication de fascicule du brevet: **15.12.93**

(51) Int. Cl.5: **C07C 2/66**, C07C 15/02, C07C 15/085, B01J 29/18

(21) Numéro de dépôt: **89402774.7**

(22) Date de dépôt: **09.10.89**

Le dossier contient des informations techniques présentées postérieurement au dépôt de la demande et ne figurant pas dans le présent fascicule.

(54) **Procédé de production d'alkylbenzènes utilisant un catalyseur à base de mordénite modifiée.**

(30) Priorité: **26.10.88 FR 8814099**

(43) Date de publication de la demande:
**02.05.90 Bulletin 90/18**

(45) Mention de la délivrance du brevet:
**15.12.93 Bulletin 93/50**

(84) Etats contractants désignés:
**BE CH DE ES FR GB IT LI NL**

(56) Documents cités:
EP-A- 0 029 652     FR-A- 2 084 704
US-A- 3 716 597     US-A- 3 849 340
US-A- 4 008 290     US-A- 4 237 329

CHEMICAL ABSTRACTS, vol. 100, no. 20, 14 mai 1984, page 152, colonne 1, abrégé no. 159167q, Columbus, Ohio, USA; S. B. KOGAN et al: "Formation of alkylaromatic hydrocarbons in the reaction of paraffins with benzene on H-mordenite"

(73) Titulaire: **INSTITUT FRANCAIS DU PETROLE**
**4, Avenue de Bois Préau**
**F-92502 Rueil-Malmaison(FR)**

(72) Inventeur: **Juguin, Bernard**
**46, Avenue du Stade**
**F-92500 Rueil Malmaison(FR)**
Inventeur: **Raatz, Francis**
**10, Allée Jacques Prévert**
**F-78260 Achères(FR)**
Inventeur: **Travers, Christine**
**12, Boulevard du Général De Gaulle**
**F-92500 Rueil Malmaison(FR)**
Inventeur: **Martino, Germain**
**Bâtiment Condé**
**80, Avenue F. Lefèbvre**
**F-78300 Poissy(FR)**

EP 0 366 515 B1

CHEMICAL ABSTRACTS, vol 78, no. 14, 9 avril 1973, page 318, colonne 1, abrégé no. 88974q, Columbus, Ohio, USA; K. A. BECKER et al: "Benzene alkylation with ethylene and propylene over H-mordenite as catalyst"

CHEMICAL ABSTRACTS, vol. 107, no. 24, 14 décembre 1987, page 101, colonne 2, abrégé no. 219441f, Columbus, Ohio, USA; K. M. MI-NACHEV et al: "Catalytice alkylation of benzene with ethylene on high-silica zeolites of different structural types"

CHEMICAL ABSTRACTS, vol. 107, no. 6, 6 juillet 1987, page 631, colonne 1, abrégé no. 6847f, Columbus, Ohio, USA; C. F. REN et al: "Alkylation of chlorobenzene over H-mordenite and H-ZSM-5: effect of silico/aluminium ratio"

B. IMELIK et al.: CATALYSIS BY ZEOLITES, Studies in Surface Science and Catalysis, vol. 5, pages 151-159, 1980, Elsevier Scientific Publishing Company, Amsterdam, NL; H. KARGE et al: "Hydrocarbon reactions catalysed by mordenites"

## Description

La présente invention concerne un procédé de production d'au moins un alkylbenzène par alkylation du benzène au moyen de mono-oléfine(s). La mono-oléfine ou le mélange de mono-oléfines de départ peut provenir de toute source connue, par exemple d'unités de vapocraquage ou de craquage catalytique.

On a déjà proposé, pour effectuer cette réaction, l'emploi de zéolithes sous forme H, en particulier la ZSM5 (EP - 0.104.729).

Plus récemment, d'autres zéolithes ont été mises en avant, en particulier certaines mordénites, seules ou en mélange avec de la faujasite (US 3 436 432), éventuellement avec un métal du groupe VIII déposé dessus (US 3 851 004). Des brevets japonais JP 58216128 et JP 58159427 préconisent des mordénites seules, de faible rapport Si/Al (4,5 à 5), contenant des ions métalliques tels que Mg, K, Ca, ou sous forme $H^+$ (JP 041670).

Le brevet FR-A-2.084.704 décrit une composition catalytique de type mordénite désaluminée, utilisable en alkylation du benzène, la mordénite ayant un rapport Si/Al compris entre 25 et 50.

Le brevet EP-A-0.029.652 décrit un procédé d'alkylation de benzène par une mono-oléfine à longue chaîne (telle que l'octène) en présence d'une mordénite désaluminée Si/Al = 46,5 présentant des caractéristiques poreuses particulières de façon à favoriser la production du dérivé 2-phénylalcane.

Dans un brevet US 3.849.340 une composition catalytique comprenant une mordénite désaluminée (Si/Al = 25 à 50) et un métal associé stabilisant (Ag, Cu, Au, Zr) est utilisée pour des réactions d'alkylation ou transalkylation.

L'invention a pour objet d'améliorer le rendement d'alkylation en mono-alkylbenzènes, en réduisant la proportion des poly-alkylbenzènes formés.

Selon ce procédé, on procède à l'alkylation du benzène avec une charge contenant au moins une mono-oléfine aliphatique en présence d'au moins un catalyseur à base de mordénite désaluminée, ladite mordénite présentant un rapport Si/Al compris entre plus que 50 et 80, puis on fractionne le mélange issu de l'alkylation en une première fraction contenant du benzène non converti et au moins un mono-alkylbenzène et une seconde fraction renfermant au moins un polyalkybenzène, et on fait réagir au moins une partie de ladite seconde fraction avec du benzène au contact d'au moins un catalyseur à base d'une mordénite désaluminée de rapport Si/Al compris entre plus que 50 et 80 pour recueillir au moins un mono-alkylbenzène.

Ainsi, on fait d'abord réagir (première étape ou étape d'alkylation) du benzène avec une charge renfermant au moins une mono-oléfine aliphatique au contact d'au moins un catalyseur à base d'une mordénite désaluminée, de rapport atomique Si/Al global compris entre plus que 50 et 80, de préférence entre 60 et 80, on fractionne le produit de manière à recueillir séparément une première fraction renfermant du benzène non converti et au moins un mono-alkylbenzène et une seconde fraction polyalkylbenzène (c'est-à-dire une fraction renfermant au moins un polyalkylbenzène).

Dans une seconde étape (étape de transalkylation), on fait réagir au moins une partie de ladite fraction polyalkylbenzène avec du benzène, par exemple avec du benzène dont au moins une partie est constituée de benzène non-converti dans la première étape (c'est-à-dire de benzène non-converti provenant de ladite première fraction obtenue dans la première étape) ou par exemple avec du benzène dont au moins une partie n'est pas constituée de benzène non-converti dans la première étape, au contact d'au moins un catalyseur à base d'une mordénite désaluminée de rapport atomique Si/Al global compris entre plus que 50 et 80, de préférence entre 60 et 80, et on recueille au moins un mono-alkylbenzène (le catalyseur de la seconde étape étant en général distinct de celui de la première étape).

Le benzène excédentaire de la première étape qui n'est pas envoyé à la seconde étape est avantageusement recyclé à la première étape, tandis que, après la seconde étape, les polyalkylbenzènes non convertis peuvent être recyclés dans cette même seconde étape.

Ainsi, les mono-alkylbenzènes obtenus par la variante préférée en deux étapes du procédé selon l'invention proviennent, d'une part, de la première fraction obtenue à la première étape (cette fraction pouvant être elle-même fractionnée en benzène non-converti et en au moins un mono-alkylbenzène), et, d'autre part, de la réaction (seconde étape) de la seconde fraction obtenue à la première étape avec du benzène.

La mordénite désaluminée est employée seule ou en mélange avec un liant ou une matrice généralement choisis dans le groupe formé par les argiles, les alumines, la silice, la magnésie, la zircone, l'oxyde de titane, l'oxyde de bore et toute combinaison d'au moins deux des composés précités comme la silice-alumine, la silice-magnésie etc... Toutes les méthodes connues d'agglomération et de mise en forme sont applicables, telles que, par exemple, l'extrusion, le pastillage, la coagulation en gouttes etc...

3

On utilise ainsi dans le procédé selon l'invention au moins un catalyseur à base de mordénite désaluminée de rapport atomique Si/Al global compris entre plus que 50 et 80, de préférence entre 60 et 80, contenant généralement 1 à 100 %, de préférence 20 à 98 % et, par exemple, 40 à 98 % en poids de ladite mordénite désaluminée et 0 à 99 %, de préférence 2 à 80 % et, par exemple, 2 à 60 % en poids d'une matrice.

Les mordénites désaluminées et leur préparation sont connues. On pourra par exemple se référer aux brevets européens EP-0084748, EP-0097552 et EP-0196965 et à la demande de brevet français de numéro d'enregistrement national 87/12932. La méthode de désalumination des brevets ou de la demande de brevet précités consiste à soumettre la forme H de la mordénite ou un précurseur de forme H (par exemple la forme $NH_4$) à une série de chauffages en atmosphère de vapeur d'eau et de traitements acides. Toutefois, par différence avec les traitements desdits documents précités, on limitera ici le taux de désalumination à un rapport atomique Si/Al global entre plus que 50 et 80. Le brevet EP-0084748, page 4, décrit d'ailleurs des préparations aboutissant à des rapports atomiques Si/Al globaux de 46 et 82 par exemple. On opère de préférence comme suit :

- dans une première étape, on élimine les cations non décomposables, généralement $Na^+$, présents dans la mordénite de départ. Pour ce faire, on pourra procéder à un ou plusieurs échanges dans des solutions diluées d'acides comme HCl ou dans des solutions de $NH_4^+$, éventuellement suivis d'un ou plusieurs lavages (à l'eau par exemple). Le point important est que à l'issue de cette première étape que l'on peut qualifier de décationisation, la quasi-totalité des cations alcalins soit éliminée (teneur en Na par exemple comprise entre 150 et 2000 ppm en poids et, de préférence, entre 300 et 1200 ppm en poids) et le solide obtenu soit une forme H ou un précurseur de forme H (exemple $NH_4^+$) non désaluminée substantiellement (taux de désalumination généralement inférieur à 10 % et de préférence à 5 %). De manière préférée, on choisira, comme précurseur de forme H, la forme $NH_4^+$ ;
- dans une deuxième étape, la forme H ou le précurseur de forme H est soumis à un traitement sous vapeur d'eau à une température supérieure à 450°C, par exemple comprise entre 450 et 650°C et, de préférence, entre 550 et 600°C. La teneur en eau (en volume) de l'atmosphère de calcination sera avantageusement supérieure à 20 % et, de manière préférée, à 40 %;
- l'attaque acide constitue la troisième étape de la préparation des catalyseurs. Pour des rapports atomiques Si/Al de charpente (du solide après calcination) jusqu'à 50 environ, on utilisera de préférence des concentrations de solutions acides (HCl, $H_2SO_4$, $HNO_3$, etc...) comprises entre 0,5 et 5 N et, de préférence, entre 1 et 4 N. Pour des rapports atomiques Si/Al de charpente supérieurs, on utilisera des concentrations de solutions acides comprises entre 5 et 20 N et de préférence entre 7 et 12 N (les rapports atomiques Si/Al de charpente peuvent être déterminés par spectroscopie infra-rouge pour des rapports compris entre 10 et 50 et par RMN du $^{29}$Si pour des rapports supérieurs).
  Par ailleurs, pour atteindre des rapports atomiques Si/Al élevés, c'est-à-dire supérieurs à environ 50 et plus spécifiquement supérieurs à environ 60, on pourra recourir avantageusement à plusieurs cycles calcination sous vapeur d'eau-attaque acide.

Les solides ainsi préparés ont avantageusement des rapports atomiques Si/Al globaux de 30 à 80, de préférence entre 40 et 80 ; ils ont un volume de maille élémentaire situé entre 2,755 et 2,730 $nm^3$ (1nm = $10^{-9}$ m) et, de préférence, entre 2,745 et 2,735 $nm^3$ ; ils ont de préférence une force acide suffisante pour que les Al-OH structuraux interagissent avec une base faible comme l'éthylène (mesure infra-rouge à 77 K) ou un composé à caractère faiblement acide comme $H_2S$ (mesure infra-rouge à 25°C). Ces solides doivent, en outre, être de préférence exempts d'espèces cationiques extra-réseau que l'on peut détecter par un signal fin (largeur à mi hauteur inférieure à 5 ppm et de préférence inférieure à 2 ppm) situé à 0 ppm (référence $Al(H_2O)_6^{3+}$) sur un spectre de RMN de $^{27}$Al, mesuré avec la technique de rotation de l'angle magique.

La réaction dite d'alkylation est habituellement effectuée en phase liquide, en phase supercritique ou en phase gazeuse, en présence d'au moins un catalyseur à base de la mordénite désaluminée définie plus haut, disposé en lit fixe, à une température d'environ 50 à 450°C (de préférence d'environ 100 à 350°C), sous une pression de 1 à 10 MPa (de préférence de 2 à 7 MPa), avec un débit d'hydrocarbures liquides (vitesse spatiale) d'environ 0,5 à 50 volumes par volume de catalyseur et par heure, et avec un rapport molaire benzène/mono-oléfines compris entre 1 et 20 (de préférence, entre 3 et 7 pour le propène et entre 5,5 et 12 pour l'éthylène).

La réaction dite de transalkylation des polyalkylbenzènes formés au cours de l'étape d'alkylation est habituellement effectuée en présence d'au moins un catalyseur à base de la mordénite désaluminée définie plus haut, disposé en lit fixe, à une température d'environ 200 à 500°C (de préférence d'environ 280 à 400°C), sous une pression de 2 à 10 MPa (de préférence entre 3 et 7 MPa), avec un débit d'hydrocarbures liquides (vitesse spatiale) d'environ 0,5 à 5 volumes par volume de catalyseur et par heure, avec un rapport molaire benzène/polyalkylbenzènes de 0,2 à 10.

L'étape de transalkylation peut éventuellement s'effectuer en présence d'hydrogène (rapport molaire hydrogène/hydrocarbures compris par exemple entre 2 et 6).

Bien que l'on puisse mettre en oeuvre l'invention avec un mélange de mono-oléfines, par exemple un effluent de craquage catalytique, on préfère, afin de faciliter le fractionnement entre mono-alkylbenzènes et poly-alkylbenzènes lorsqu'on opère avec une transalkylation subséquente, opérer avec une seule mono-oléfine, par exemple l'éthylène, le propylène, un n-butène ou l'isobutène, plus généralement avec une mono-oléfine aliphatique ayant par exemple de 2 à 20 atomes de carbone.

Le traitement de mélanges de mono-oléfines renfermant du monoxyde de carbone, par exemple 0,1 à 2,0 % en poids, ce qui est souvent le cas des effluents de cracking, présente un intérêt particulier. En effet la présence du monoxyde de carbone n'a pas d'effet néfaste, contrairement à ce qui est observé avec les catalyseurs conventionnels renfermant du nickel.

Quand on utilise une transalkylation, pour simplifier la mise en oeuvre de l'invention, on peut utiliser une zone de distillation unique pour les effluents des deux étapes éventuelles du procédé (alkylation et transalkylation) selon toute technique bien connue de l'homme du métier.

Les exemples suivants illustrent l'invention sans en limiter la portée.

EXEMPLE 1A : Préparation de trois catalyseurs B1, B2, B3.

La matière première utilisée pour préparer ces divers catalyseurs est une mordénite petits pores de la Société Chimique de la Grande Paroisse, référencée Alite 150 ; sa formule chimique à l'état anhydre est $Na, AlO_2(SiO_2)_{5,5}$ et sa teneur pondérale en sodium de 5,3 %. 500 grammes de cette poudre sont plongés dans une solution 2 M de nitrate d'ammonium, et la suspension est portée à 95°C pendant 2 heures. Le volume de la solution de nitrate d'ammonium engagée est égal à 4 fois le poids de mordénite sèche (V/P = 4 $g/cm^3$). Cette opération d'échange cationique est recommencée 3 fois. Après le 3ème échange, le produit est lavé à l'eau à 20°C pendant 20 minutes, avec un rapport V/P égal à 4 $g/cm^3$. La teneur en sodium exprimée en pourcentage en poids par rapport à la mordénite sèche n'est plus que de 0,1 %. Le produit est ensuite filtré, et différents lots sont soumis à une calcination sous vapeur d'eau en atmosphère confinée ("self-steaming"), à une température plus ou moins élevée suivant le degré de désalumination de la charpente que l'on veut obtenir (tableau 1). La durée de la calcination est fixée à 2 heures. La teneur en eau (en volume) de l'atmosphère de calcination est de l'ordre de 90 %. La cristallinité des différents solides, après cette étape de calcination, est supérieure ou égale à 90 %.

On procède ensuite, sur chacun des solides, à une attaque acide à l'aide d'une solution d'acide nitrique de concentration d'autant plus élevée que le taux de désalumination de la charpente obtenu, lors de l'étape précédente, est plus important (tableau 1). Au cours de l'attaque acide, le solide est ainsi porté à reflux dans la solution d'acide nitrique pendant 2 heures, avec un rapport V/P égal à 8 $g/cm^3$. Le produit est ensuite filtré, puis lavé abondamment à l'eau distillée.

Les rapports atomiques Si/Al obtenus pour chacune des mordénites figurent dans le tableau 1.

Chaque mordénite est ensuite mise en forme par malaxage avec de l'alumine, puis par passage au travers d'une filière. Les extrudés obtenus, de diamètre 1,2 mm, sont ensuite séchés puis calcinés entre 150 et 500°C par palier d'une heure environ. On obtient alors des catalyseurs B1, B2 et B3 à base de mordénite, contenant 80 % en poids de mordénite et 20 % en poids d'alumine.

TABLEAU 1

| CATALYSEUR | B1 | B2 | B3 |
|---|---|---|---|
| Température de calcination (°C) | 510 | 560 | 600 |
| Rapport atomique Si/Al global* de la mordénite | 5,5 | 5,5 | 5,5 |
| Rapport atomique Si/Al IV de charpente de la mordénite | 11 | 49 | 86 |
| Normalité de la solution d'acide nitrique | 1N | 3,5N | 10N |
| Rapport atomique Si/Al global** de la mordénite | 11 | 49 | 86 |

* après calcination
** après attaque acide.

EP 0 366 515 B1

EXEMPLE 1B : préparation de 3 catalyseurs B'1,B'2, B'3.

Ces catalyseurs diffèrent de ceux décrits dans l'exmple 1A, en ce que la matière première utilisée pour les préparer n'est plus la mordénite Alite 150 de la Société Chimique de la Grande Paroisse, mais une mordénite larges pores de la Société TOYO-SODA, référencée TSZ 600 NAA. Sa formule chimique, à l'état anhydre, est Na, $AlO_2(SiO_2)_{5,1}$ et sa teneur pondérale en sodium de 5,7 %.

Toutes les étapes d'échange, de calcination, d'attaque acide, de mise en forme et de calcination, sont effectuées dans les mêmes conditions que celles décrites dans l'exemple 1A. Les rapports Si/Al atomiques obtenus ne sont que très légèrement différents (tableau 2).

Les catalyseurs obtenus (B′1, B′2 et B′3) contiennent également 80 % en poids de mordénite et 20 % en poids d'alumine.

TABLEAU 2

| CATALYSEUR | B'1 | B'2 | B'3 |
|---|---|---|---|
| Température de calcination (°C) | 510 | 560 | 600 |
| Rapport atomique Si/Al global* de la mordénite | 5,1 | 5,1 | 5,1 |
| Rapport atomique Si/Al IV de charpente de la mordénite | 10 | 47 | 83 |
| Normalité de la solution d'acide nitrique | 1 N | 3,5 N | 10 N |
| Rapport atomique Si/Al global** de la mordénite | 10 | 47 | 83 |

\* après calcination
\** après attaque acide.

EXEMPLE 2A :

Les trois catalyseurs B1, B2, B3, préparés dans l'exemple 1A, sont chacun testés en alkylation du benzène par le propène dans les conditions opératoires suivantes (dans un réacteur dit "réacteur d'alkylation") :
- température : 160°C ;
- pression : 4,5 MPa ;
- débit horaire pondéral de benzène égal à 2,5 fois le poids du catalyseur ;
- rapport molaire benzène/propène = 5.
La charge a la composition pondérale suivante :

| propane | 0,22 % |
|---|---|
| propène | 9,78 % |
| benzène | 90 % |

A la sortie du réacteur, les produits obtenus avec chacun des catalyseurs B1, B2 et B3 ont la composition pondérale présentée dans le tableau 3. Chaque produit est alors fractionné par distillation, et deux fractions sont recueillies :
- une fraction point initial - 160°C contenant le benzène non-converti et le cumène,
- une fraction de point d'ébullition supérieur à 160°C contenant les polyisopropylbenzènes et les hydrocarbures (ou HC) aromatiques lourds.
Chaque fraction de point d'ébullition supérieur à 160°C subit ensuite un traitement de transalkylation en présence du même catalyseur B1, B2 ou B3 (distinct de celui utilisé en alkylation) dans les conditions opératoires suivantes (dans un réacteur dit "réacteur de transalkylation") :
- température 330°C
- pression 4,5 MPa
- débit pondéral de charge (polyisopropylbenzènes + hydrocarbures aromatiques lourds + benzène additionnel (qui provient du produit de la réaction d'alkylation)) égal à 2,5 fois le poids du catalyseur.

6

Les résultats obtenus (par le traitement de transalkylation-désalkylation)sont présentés dans le tableau 4.

Les résultats d'ensemble combinant les deux étapes d'alkylation d'une part et de transalkylation d'autre part sont présentés dans le tableau 5.

On poursuit ensuite les essais pendant une longue durée afin de vérifier l'évolution de performances en fonction du temps ; les résultats d'ensemble combinant les deux étapes sont présentés dans le tableau 6.

A l'examen des résultats, on peut constater qu'il est préférable de travailler avec les catalyseurs préconisés par l'invention (aussi bien en ce qui concerne les résultats d'ensemble, qu'en ce qui concerne les résultats particuliers obtenus dans les deux étapes du procédé), c'est-à-dire qu'il faut utiliser des mordénites désaluminées ayant un rapport atomique Si/Al global compris entre 30 et 80, de préférence entre 40 et 80 (cas du catalyseur B2). En effet, les mordénites ayant un rapport atomique Si/Al global inférieur à 30 (cas du catalyseur B1) sont légèrement plus actives, mais nettement moins sélectives et peu stables. En revanche, les mordénites ayant un rapport atomique Si/Al global supérieur à 80 (cas du catalyseur B3) sont un peu plus sélectives, mais beaucoup moins actives et de stabilité inférieure.

En ce qui concerne la mise en oeuvre industrielle de ce type de procédé, les diisopropylbenzènes et les triisopropylbenzènes non transformés après l'étape de transalkylation sont recyclés à extinction dans le réacteur de transalkylation ; dans ce cas, en utilisant le catalyseur B2 (dans les 2 étapes) les performances ultimes sont les suivantes :

1) Taux de conversion :
- benzène : 18,7 % ;
- propène : 100 %.
2) Sélectivités :
- cumène par rapport au benzène transformé : 99,4 % ;

7

- cumène par rapport au propène transformé : 93,2 %.

## TABLEAU 3

| CATALYSEURS \ CONSTITUANTS (% poids) | CHARGE | B1 | B2 | B3 |
|---|---|---|---|---|
| Méthane | – | 0,20 | – | – |
| Ethane | – | 0,22 | – | – |
| Propane | 0,22 | 0,65 | 0,22 | 0,22 |
| Propène | 9,78 | – | – | 2,09 |
| Butanes | – | 0,09 | – | – |
| Benzène | 90 | 74,91 | 73,61 | 76,35 |
| Cumène | – | 21,34 | 23,22 | 20,25 |
| Diisopropylbenzènes | – | 1,08 | 1,89 | 0,76 |
| Triisopropylbenzènes | – | 1,01 | 0,66 | 0,20 |
| HC aromatiques lourds | – | 0,50 | 0,40 | 0,13 |
| | 100 | 100 | 100 | 100 |
| **TAUX DE CONVERSION PAR PASSE** | | | | |
| Propène | | 100 % | 100 % | 78,6 % |
| Benzène | | 16,8 % | 18,2 % | 15,2 % |
| **SELECTIVITES** | | | | |
| $\dfrac{\text{Cumène}}{\text{Propène transformé}} \times 100$ | | 76,4 % | 83,1 % | 92,2 % |
| $\dfrac{\text{Cumène}}{\text{Benzène transformé}} \times 100$ | | 91,9 % | 92,1 % | 96,4 % |

EP 0 366 515 B1

TABLEAU 4

| CATALYSEURS<br><br>CONSTITUANTS<br>(% poids) | B1 | B2 | B3 |
|---|---|---|---|
| **CHARGE** | | | |
| Hydrogène | 0,22 | 0,27 | 0,10 |
| Benzène | 1,09 | 1,31 | 0,49 |
| Diisopropylbenzènes | 1,08 | 1,89 | 0,76 |
| Triisopropylbenzènes | 1,01 | 0,66 | 0,20 |
| HC aromatiques lourds | 0,50 | 0,40 | 0,13 |
| | 3,90 | 4,53 | 1,68 |
| **PRODUIT** | | | |
| Hydrogène | 0,22 | 0,27 | 0,10 |
| Méthane | 0,01 | – | – |
| Ethane | 0,02 | 0,02 | – |
| Propane | 0,12 | 0,03 | 0,01 |
| Butanes | 0,01 | – | – |
| Benzène | 0,76 | 0,97 | 0,39 |
| n propylbenzène | 0,02 | – | – |
| Cumène | 1,93 | 2,18 | 0,66 |
| Triméthylbenzènes | 0,05 | – | – |
| Diisopropylbenzènes | 0,03 | 0,32 | 0,28 |
| Triisopropylbenzènes | 0,18 | 0,28 | 0,11 |
| HC aromatiques lourds | 0,55 | 0,40 | 0,13 |
| | 3,90 | 4,53 | 1,68 |
| **TAUX DE CONVERSION PAR PASSE** | | | |
| Benzène | 30,3 % | 26 % | 20,4 % |
| Diisopropylbenzènes | 97,2 % | 83,1 % | 63,2 % |
| Triisopropylbenzènes | 82,2 % | 57,6 % | 45 % |
| **SELECTIVITE** $\dfrac{\text{Cumène formé}}{\text{benzène+diisopropylbenzènes+triisopropylbenzènes transformés}} \times 100$ | 87,3 % | 95,2 % | 98,5 % |

9

## TABLEAU 5

| CATALYSEURS<br>RESULTATS | B1 | B2 | B3 |
|---|---|---|---|
| **REACTIFS TRANSFORMES (% poids)** | | | |
| Benzène | 15,42 | 16,73 | 13,75 |
| Propène | 9,78 | 9,78 | 7,69 |
| TOTAL | 25,20 | 26,51 | 21,44 |
| **PRODUITS FORMES (% poids)** | | | |
| Méthane | 0,21 | – | – |
| Ethane | 0,24 | 0,02 | – |
| Propane | 0,55 | 0,09 | 0,01 |
| Butanes | 0,10 | – | – |
| n propylbenzène | 0,02 | – | – |
| Cumène | 23,27 | 25,40 | 20,91 |
| Triméthylbenzènes | 0,05 | – | – |
| Diisopropylbenzènes | 0,03 | 0,32 | 0,28 |
| Triisopropylbenzènes | 0,18 | 0,28 | 0,11 |
| HC aromatiques lourds | 0,55 | 0,40 | 0,13 |
| TOTAL | 25,20 | 26,51 | 21,44 |
| **TAUX DE CONVERSION** | | | |
| Benzène | 17,1 % | 18,6 % | 15,3 % |
| Propène | 100 % | 100 % | 78,6 % |
| **SELECTIVITES** | | | |
| 1) Cumène formé par rapport au benzène transformé | 98,1 % | 98,7 % | 98,9 % |
| 2) Cumène formé par rapport au propène transformé | 83,3 % | 90,9 % | 95,2 % |

TABLEAU 6

| ACE DU CATALYSEUR EN HEURES / RESULTATS | 24 | 144 | 528 | 1296 |
|---|---|---|---|---|
| **CATALYSEUR B1** | | | | |
| 1) TAUX DE CONVERSION | | | | |
| Benzène | 17,1 % | 16,6 % | 16 % | 7,6 % |
| Propène | 100 % | 100 % | 99,2 % | 78,8 % |
| 2) SELECTIVITES | | | | |
| Cumène formé par rapport au benzène transformé | 98,1 % | 98,4 % | 98,8 % | 99,3 % |
| Cumène formé par rapport au propène transformé | 83,3 % | 83,9 % | 84,2 % | 85,2 % |
| **CATALYSEUR B2** | | | | |
| 1) TAUX DE CONVERSION | | | | |
| Benzène | 18,6 % | 18,5 % | 18,4 % | 18,2 % |
| Propène | 100 % | 100 % | 100 % | 100 % |
| 2) SELECTIVITES | | | | |
| Cumène formé par rapport au benzène transformé | 98,7 % | 98,9 % | 99,1 % | 99,4 % |
| Cumène formé par rapportau propène transformé | 90,9 % | 91 % | 91,4 % | 92,2 % |
| **CATALYSEUR B3** | | | | |
| 1) TAUX DE CONVERSION | | | | |
| Benzène | 15,3 % | 15,1 % | 14,5 % | 13,2 % |
| Propène | 78,6 % | 77,9 % | 76,9 % | 72 % |
| 2) SELECTIVITES | | | | |
| Cumène formé par rapport au benzène transformé | 98,9 % | 99 % | 99,2 % | 99,5 % |
| Cumène formé par rapport au propène transformé | 95,2 % | 95,6 % | 95,8 % | 96,6 % |

EXEMPLE 2B :

Les trois catalyseurs B'1, B'2, B'3, préparés dans l'exemple 1B, sont également chacun testés dans les mêmes conditions opératoires et avec la même charge que les catalyseurs B1, B2 et B3, les résultats d'ensemble combinant les deux étapes du procédé (alkylation et transalkylation) sont présentés dans le tableau 7.

EP 0 366 515 B1

## TABLEAU 7

| CATALYSEURS RESULTATS | B'1 | B'2 | B'3 |
|---|---|---|---|
| TAUX DE CONVERSION | | | |
| Benzène | 17,8 % | 18,9 % | 16,5 % |
| Propène | 100 % | 100 % | 83,8 % |
| SELECTIVITES | | | |
| 1) Cumène formé par rapport au benzène transformé | 94,6 % | 98,5 % | 98,7 % |
| 2) Cumène formé par rapport au propène transformé | 81,7 % | 90,7 % | 95,9 % |

Comme dans le cas de l'exemple 2A, on peut constater qu'il est préférable de travailler avec des catalyseurs préconisés par l'invention, c'est-à-dire qu'il faut utiliser des mordénites dont le rapport atomique Si/Al global est compris entre 30 et 80, de préférence entre 40 et 80.

EXEMPLE 3 :

En utilisant le catalyseur B2 de l'exemple 1A et les conditions opératoires de l'exemple 2A dans les deux sections d'alkylation et de transalkylation, on fait varier le rapport molaire benzène/propène à l'entrée du réacteur d'alkylation, afin de montrer l'importance de ce paramètre sur l'activité et surtout sur la sélectivité du catalyseur. Les résultats d'ensemble combinant les deux étapes d'alkylation d'une part et de transalkylation d'autre part sont présentés dans le tableau 8.

On peut constater la grande influence de ce paramètre sur la sélectivité du catalyseur ; comme préconisé dans le cadre de l'invention, il est préférable de travailler avec un rapport molaire benzène/propène compris entre 3 et 7. En effet pour des rapports molaires benzène/propène inférieurs à 3, la sélectivité est relativement basse. Pour des rapports molaires benzène/propène supérieurs à 7, si la sélectivité est bonne, il est nécessaire d'adopter des taux de recyclage de benzène très élevés, ce qui grève fortement l'économie globale du procédé.

12

# EP 0 366 515 B1

TABLEAU 8

| RESULTATS ⟍ BENZENE ——— molaire PROPENE | 1,11 | 2,49 | 4,96 | 7,92 |
|---|---|---|---|---|
| TAUX DE CONVERSION | | | | |
| Benzène | 71 % | 35,1 % | 18,6 % | 12,4 % |
| Propène | 100 % | 100 % | 100 % | 100 % |
| SELECTIVITES | | | | |
| 1) Cumène formé par rapport au benzène transformé | 94,2 % | 97,5 % | 98,7 % | 99,3 % |
| 2) Cumène formé par rapport au propène transformé | 74,4 % | 85,3 % | 90,9 % | 97,5 % |

EXEMPLE 4 :

Les trois catalyseurs B1, B2, B3, préparés dans l'exemple 1A, sont chacun testés en alkylation du benzène par l'éthylène dans les conditions opératoires suivantes (dans un réacteur dit "réacteur d'alkylation") :
- température : 200 °C ;
- pression : 4,5 MPa ;
- débit pondéral de benzène égal à 2,5 fois le poids du catalyseur ;
- rapport molaire benzène/éthylène = 6,7.
La charge a la composition pondérale suivante :

| éthylène | 5,07 % |
|---|---|
| benzène | 94,93 %. |

A la sortie du réacteur, chaque produit obtenu est fractionné, et deux fractions sont recueillies :
- une fraction point initial - 140 °C contenant le benzène non-converti et l'éthylbenzène,
- une fraction de point d'ébullition supérieur à 140 °C contenant les polyéthylbenzènes et les hydrocarbures (ou HC ) aromatiques lourds.
Chaque fraction de point d'ébullition supérieur à 140 °C subit ensuite un traitement de transalkylation en présence du même catalyseur B1, B2 ou B3 (distinct de celui utilisé en alkylation) dans les conditions opératoires suivantes (dans un réacteur dit "réacteur de transalkylation") :
- température : 370 °C ;
- pression : 4,5 MPa ;
- débit pondéral de charge (polyéthylbenzènes + hydrocarbures aromatiques lourds + benzène additionnel (qui provient du produit de la réaction d'alkylation)) égal à 1,4 fois le poids du catalyseur.
Les résultats d'ensemble combinant les deux étapes d'alkylation d'une part et de transalkylation d'autre part sont présentés dans le tableau 9.

13

TABLEAU 9

| CATALYSEURS RESULTATS | B1 | B2 | B3 |
|---|---|---|---|
| **REACTIFS TRANSFORMES (% poids)** | | | |
| Benzène | 13,02 | 11,90 | 6,99 |
| Ethylène | 5,07 | 5,07 | 3,04 |
| Total | 18,09 | 16,97 | 10,03 |
| **TAUX DE CONVERSION** | | | |
| Benzène | 13,7 % | 12,5 % | 7,4 % |
| Ethylène | 100 % | 100 % | 60 % |
| **SELECTIVITES** | | | |
| 1) Ethylbenzène formé par rapport au benzène transformé | 80,6 % | 94,8 % | 96 % |
| 2) Ethylbenzène formé par rapport à l'éthylène transformé | 74,3 % | 79,9 % | 79,2 % |

EXEMPLE 5 :

Les trois catalyseurs B1, B2, B3, préparés dans l'exemple 1A, sont chacun testés en alkylation du benzène par des gaz issus d'une unité de craquage catalytique ; ces gaz ont la composition suivante (% poids) :
- hydrogène : 0,46
- monoxyde de carbone (CO) : 4,49
- dioxyde de carbone ($CO_2$) : 0,36
- azote : 15,09
- oxygène : 0,26
- méthane : 23,18
- éthane : 23,62
- éthylène : 16,50
- propane : 2,89
- propène : 5,88
- butanes : 1,58
- butènes : 3,31
- pentanes : 0,76
- pentènes : 1,62

Les conditions opératoires sont les suivantes (dans un réacteur dit "réacteur d'alkylation") :
- température : 220 °C ;
- pression : 4,5 MPa ;

14

- débit horaire pondéral de benzène égal à 2,5 fois le poids du catalyseur ;
- rapport molaire benzène/oléfines (C2 + C3 + C4 + C5) = 7,2.

A la sortie du réacteur d'alkylation, et après 24 heures de fonctionnement, on a obtenu les résultats du tableau 10.

TABLEAU 10

| CATALYSEURS | B1 | B2 | B3 |
|---|---|---|---|
| **TAUX DE CONVERSION PAR PASSE** | | | |
| Ethylène | 100 % | 100 % | 60 % |
| Propène | 100 % | 100 % | 88,6 % |
| Butènes | 100 % | 100 % | 93 % |
| Pentènes | 100 % | 100 % | 87 % |
| Benzène | 8,6 % | 11,8 % | 8,5 % |
| **SELECTIVITES** | | | |
| $\dfrac{\text{éthylbenzène + cumène}}{\text{éthylène + propène transformés}} \times 100$ | 47,9 % | 70,4 % | 79,3 % |
| $\dfrac{\text{éthylbenzène + cumène}}{\text{benzène transformé}} \times 100$ | 80,4 % | 84,6 % | 89,6 % |

Le produit obtenu avec le catalyseur B2 est ensuite fractionné par distillation, et deux fractions ont été recueillies :
- une fraction point initial - 160°C contenant le benzène non-converti, l'éthylbenzène et le cumène,
- une fraction 160°C- point final contenant les alkylbenzènes supérieurs et les polyalkylbenzènes ; cette fraction présente un point 95 % d'ébullition ASTM égal à 224°C ; ses indices d'octane mesurés au moteur CFR ont les valeurs suivantes :

| RON clear | 124 |
|---|---|
| MON clear | 107. |

**Revendications**

1. Procédé de production d'au moins un alkylbenzène, dans lequel on procède à l'alkylation du benzène avec une charge contenant au moins une mono-oléfine aliphatique en présence d'au moins un catalyseur à base de mordénite désaluminée, caractérisé en ce que ladite mordénite présente un rapport Si/Al compris entre plus que 50 et 80, que le mélange issu de l'alkylation est fractionné en une première fraction contenant du benzène non-converti et au moins un mono-alkylbenzène et en une seconde fraction renfermant au moins un polyalkylbenzène, en ce qu'on fait réagir au moins une partie de ladite seconde fraction avec du benzène au contact d'au moins un catalyseur à base d'une mordénite désaluminée de rapport Si/Al compris entre plus que 50 et 80 et ce qu'on recueille au moins un mono-alkylbenzène.

**2.** Procédé selon la revendication 1, caractérisé en ce que la mordénite désaluminée utilisée en alkylation présente un rapport Si/Al compris entre 60 et 80.

**3.** Procédé selon l'une des revendications précédentes, caractérisé en ce que la mordénite désaluminée utilisée en transalkylation présente un rapport Si/Al compris entre 60 et 80.

**4.** Procédé selon la revendication 3 dans lequel au moins une partie du benzène avec lequel on fait réagir ladite seconde fraction est constituée de benzène non-converti provenant de ladite première fraction.

**5.** Procédé selon l'une des revendications 3 et 4 dans lequel les polyalkylbenzènes non-convertis lors de la réaction du benzène avec au moins une partie de ladite seconde fraction sont recyclés dans cette étape.

**6.** Procédé selon l'une des revendications 1 à 5 dans lequel chaque catalyseur est disposé en lit fixe.

**7.** Procédé selon l'une des revendications 1 à 6 dans lequel ladite charge renferme 0,1 à 2,0 % en poids de monoxyde de carbone.

**8.** Procédé selon l'une des revendications 1 à 7 dans lequel ladite charge est un effluent de craquage catalytique.

**9.** Procédé de production d'éthylbenzène selon l'une des revendications 1 à 8 dans lequel, lors de la réaction du benzène avec une charge renfermant de l'éthylène, le rapport molaire benzène/éthylène est compris entre 5,5 et 12.

**10.** Procédé de production de cumène selon l'une des revendications 1 à 8 dans lequel, lors de la réaction du benzène avec une charge renfermant du propène, le rapport molaire benzène/propène est compris entre 3 et 7.

## Claims

**1.** Process for the production of at least one alkyl benzene, in which the benzene is alkylated with a charge containing at least one aliphatic monoolefin in the presence of at least one catalyst based on dealuminated mordenite, characterized in that said mordenite has a Si/Al ratio between more than 50 and 80, that the mixture resulting from the alkylation is fractionated into a first fraction containing unconverted benzene and at least one monoalkyl benzene and into a second fraction containing at least one polyalkyl benzene and in that at least part of said second fraction is reacted with the benzene in contact with at least one catalyst based on a dealuminated mordenite having a Si/Al ratio between more than 50 and 80 and in that at least one monoalkyl benzene is collected.

**2.** Process according to claim 1, characterized in that the dealuminated mordenite used in alkylation has a Si/Al ratio between 60 and 80.

**3.** Process according to either of the preceding claims, characterized in that the dealuminated mordenite used in transalkylation has a Si/Al ratio between 60 and 80.

**4.** Process according to claim 3, wherein at least part of the benzene with which is reacted said second fraction is constituted by unconverted benzene coming from said first fraction.

**5.** Process according to either of the claims 3 and 4, wherein the polyalkylbenzenes not converted during the reaction of the benzene with at least part of said second fraction are recycled to said stage.

**6.** Process according to any one of the claims 1 to 5, wherein said catalyst is in fixed bed form.

7. Process according to any one of the claims 1 to 6, wherein said charge contains 0.1 to 2.0 % by weight carbon monoxide.

8. Process according to any one of the claims 1 to 7, wherein said charge is a catalytic cracking effluent.

9. Process for the production of ethyl benzene according to any one of the claims 1 to 8, wherein, during the reaction of the benzene with a charge containing ethylene, the benzene/ethylene molar ratio is between 5.5. and 12.

10. Process for the production of cumene according to any one of the claims 1 to 8, wherein, during the reaction of the benzene with a charge containing propene, the benzene/propene molar ratio is between 3 and 7.

**Patentansprüche**

1. Verfahren zur Herstellung wenigstens eines Alkylbenzoles, in welchem man die Alkylierung von Benzol mit einer Beschickung, welche wenigstens ein aliphatisches Monoolefin enthält, in Gegenwart wenigstens eines Katalysators auf Basis von entaluminiertem Mordenit durchführt, dadurch **gekennzeichnet,** daß der Mordenit ein Si/Al-Verhältnis, welches zwischen mehr als 50 und 80 liegt, zeigt, daß die aus der Alkylierung stammende Mischung in eine erste Fraktion fraktioniert wird, welche nicht-umgewandeltes Benzol und wenigstens ein Monoalkylbenzol enthält, und in eine zweite Fraktion, welche wenigstens ein Polyalkylbenzol umfaßt, in dem man wenigstens einen Teil der zweiten Fraktion mit Benzol im Kontakt mit wenigstens einem Katalysator auf Basis von entaluminiertem Mordenit mit einem Si/Al-Verhältnis, welches zwischen mehr als 50 und 80 liegt, reagieren läßt, und daß man wenigstens ein Monoalkylbenzol erhält.

2. Verfahren gemäß Anspruch 1, dadurch **gekennzeichnet,** daß der zur Alkylierung verwendete entaluminierte Mordenit ein Si/Al-Verhältnis zeigt, welches zwischen 60 und 80 liegt.

3. Verfahren gemäß einem der vorhergehenden Ansprüche, dadurch **gekennzeichnet,** daß der zur Transalkylierung verwendete entaluminierte Mordenit ein Si/Al-Verhältnis zeigt, welches zwischen 60 und 80 liegt.

4. Verfahren gemäß Anspruch 3, in welchem wenigstens ein Teil des Benzols, mit welchem man die zweite Fraktion reagieren läßt, aus nicht-umgewandeltem Benzol besteht, welches aus der ersten Fraktion stammt.

5. Verfahren gemäß einem der Ansprüche 3 und 4, in welchem die während der Reaktion von Benzol mit wenigstens einem Teil der zweiten Fraktion nicht-umgewandelten Polyalkylbenzole in diese Stufe zurückgeführt werden.

6. Verfahren gemäß einem der Ansprüche 1 bis 5, in welchem jeder Katalysator in einem Festbett angeordnet ist.

7. Verfahren gemäß einem der Ansprüche 1 bis 6, in welchem die Beschickung 0,1 bis 2,0 Gew.-% Kohlenmonoxid umfaßt.

8. Verfahren gemäß einem der Ansprüche 1 bis 7, in welchem die Beschickung ein Abstrom der katalytischen Crackung ist.

9. Verfahren zur Herstellung von Ethylbenzol gemäß einem der Ansprüche 1 bis 8, in welchem, während der Reaktion von Benzol mit einer Ethylen umfassenden Beschickung, das molare Verhältnis Benzol/Ethylen zwischen 5,5 und 12 liegt.

10. Verfahren zur Herstellung von Cumol gemäß einem der Ansprüche 1 bis 8, in welchem, während der Reaktion von Benzol mit einer Propen umfassenden Beschickung, das molare Verhältnis Benzol/Propen zwischen 3 und 7 liegt.